# EUROPEAN PATENT SPECIFICATION

(11) **EP 4 081 067 B1**
(45) Date of publication and mention of the grant of the patent: **20.12.2023**
(21) Application number: 20839071.6
(22) Date of filing: 22.12.2020
(51) Int. Cl.: A44C 25/00, A45D 40/30, A61K 8/02, A45D 44/00, A61Q 1/00, A44C 15/00, C09J 7/21

(54) **COSMETIC ARTICLE AND ASSOCIATED COSMETIC APPLICATION METHOD**
KOSMETIKARTIKEL UND DAZUGEHÖRIGES VERFAHREN ZUR KOSMETISCHEN ANWENDUNG
ARTICLE COSMÉTIQUE ET PROCÉDÉ D'APPLICATION COSMÉTIQUE ASSOCIÉ

(30) Priority: 23.12.2019 FR 1915488
(43) Date of publication of application: 02.11.2022
(73) Proprietor: L'OREAL, 75008 Paris (FR)
(72) Inventor: CAULIER, Eric, 92110 CLICHY (FR)
(74) Representative: Lavoix
(86) International application number: PCT/EP2020/087682
(87) International publication number: WO 2021/130256

(56) References cited:
- WO-A1-2013/164521
- WO-A1-2019/185162
- CN-U- 202 588 564
- FR-A1- 3 082 402
- US-A- 3 568 684
- US-A1- 2010 242 539

## Description

The present invention relates to a cosmetic article, intended to be applied on a body area of a user.

There are numerous articles, corresponding to planar and self-adhesive decorative objects, in the field of cosmetics.

However, none are fully satisfactory either in terms of hold over time, lack of flexibility, discomfort when worn, skin breathability due to an overly occlusive adhesive layer and/or decorative layer, or indeed excessive tacky effect which involves difficult and/or painful peeling.

An aim of the invention is therefore that of solving at least some of these problems, preferably all these problems.

The invention therefore relates to a cosmetic article, intended to be applied on a body area of a user, comprising a decorative layer and an adhesive layer; the decorative layer having through openings and being formed of a textile material; the adhesive layer being formed by a biocompatible adhesive, the adhesive layer being applied on one side of the decorate layer and having through openings, at least some of the through openings of the adhesive layer respectively facing the through openings of the decorative layer; the decorative layer comprising a number of through openings per dm² between 500 and 10000, and having an opening rate between 20% and 95%.

The above ranges of values of the two parameters of the number of through openings and the opening rate, combined with the fact that at least some of the through openings of the adhesive layer are respectively facing the through openings of the decorative layer, give the cosmetic article good skin breathability.

This also gives enough flexibility to fit to the different body areas on which it can be placed, and in particular movable areas such as the eyelid. Furthermore, this provides the user with comfort when worn, while ensuring a satisfactory hold over time thanks to the lightness of the article thus obtained.

The cosmetic article according to the invention may comprise one or several of the following characteristics taken in isolation or in any technically possible combination:
- the decorative layer comprises a number of through openings per dm² greater than 1500, for example between 1500 and 10000, and preferably between 1500 and 5500;
- the decorative layer has an opening rate greater than 50%, for example between 50% and 95%, and preferably between 80% and 95%;
- each through opening has a closed transverse internal contour, the transverse internal contour delimiting an internal area of the opening, the internal areas defined by the through openings of the layers being substantially identical;
- the textile material of the decorative layer is a lace;
- each through opening has a closed transverse internal contour, the transverse internal opening delimiting an internal area of the opening, at least two of the internal areas being separate, and at least one of the internal areas being preferably less than 50% of one of the other internal areas;
- each through opening of the decorative layer has a closed transverse internal contour having a greater dimension, the greater dimension of each opening being greater than 0.5 mm, and preferably less than 5 mm;
- the biocompatible adhesive comprises silicone, preferably a silicone gel;
- the adhesive layer has a mass density between 20 g/m² and 220 g/m², and preferably between 40 g/m² and 60 g/m²;
- the decorative layer has a thickness between 0.2 mm and 0.6 mm, preferably between 0.35 mm and 0.45 mm;
- the decorative layer has a mass density less than 100 g/m², more preferably less than 50 g/m², and preferably less than 10 g/m²;
- the article exhibits a force required to detach the article after 10 applications on a substrate greater than or equal to 0.16N, more preferably greater than or equal to 0.2N, in particular, between 0.2N and 0.6N, preferably between 0.2N and 0.5N;
- the article exhibits a flexural rigidity B between 0.1 µN.m²/m and 80 µN.m²/m, advantageously between 0.1 µN.m²/m and 5 µN.m²/m, preferably between 0.5 µN.m²/m and 2 µN.m²/m;
- the article exhibits a shear rigidity C between 0.1 N/(m.°) and 4 N/(m.°), advantageously between 0.1 N/(m.°) and 2 N/(m.°), preferably between 0.2 N/(m.°) and 1 N/(m.°);
- the adhesive layer comprises a number of through openings per dm² greater than or equal to 1500, for example between 1500 and 10000, and preferably between 1500 and 5500;
- at least one of the through openings of the decorative layer, referred to as "major opening", has a greater dimension greater than 5 mm, preferably greater than 10 mm, more preferably greater than 20 mm or more; and
- the or each major opening is bordered, preferably surrounded, by an area of the decorative layer having through openings wherein the greater dimension is less than that of the major opening, and for example less than 2 mm, more preferably less than 1 mm.

The invention also relates to a cosmetic application method, including the following steps:
- providing a cosmetic article as described above; and
- applying the adhesive layer on a body area of a user.

The invention will be easier to understand after reading the following description, provided solely as an example and with reference to the appended drawings, wherein:
[Fig 1] Figure 1 is a schematic perspective view of a cosmetic article according to a first embodiment;
[Fig 2] Figure 2 is a cross-sectional schematic view of the cosmetic article of figure 1;
[Fig 3] Figure 3 is a schematic perspective view of a cosmetic article according to a second embodiment;
[Fig 4] Figure 4 is a partial view of a cosmetic article according to a third embodiment; and
[Fig 5] Figure 5 is a partial view of a cosmetic article according to a fourth embodiment.

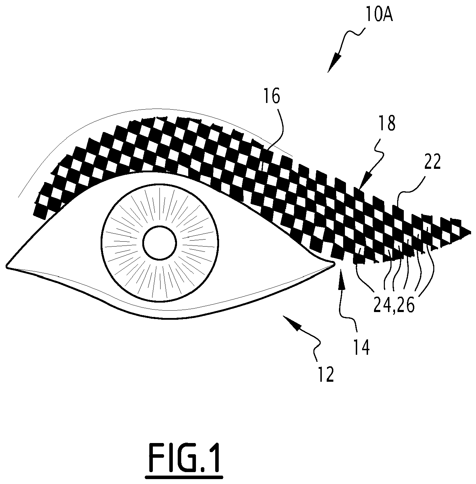

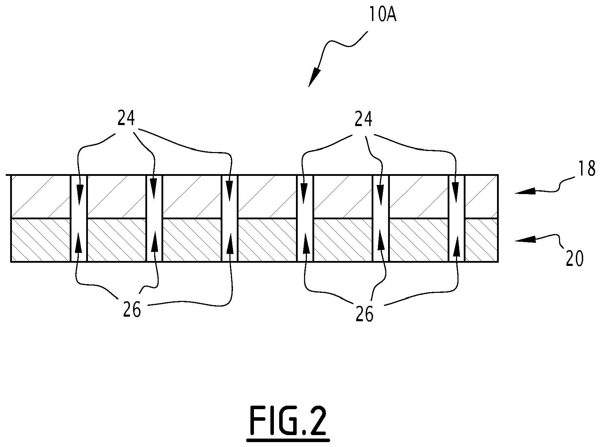

A cosmetic article 10A according to a first embodiment is illustrated in figures 1 and 2.

The cosmetic article 10A is particularly intended to be placed on a body area 12 of a user.

More specifically, the cosmetic article 10A is capable of forming a decorative object to embellish the body area 12 of the user.

The body area, on which the article 10A is placed, comprises for example a movable area and/or a stationary area.

In the example in figure 1, the body area 12 is the user's eye area, and is for example located at the outer corner 14 of the eye and/or on the upper eyelid 16 of the eye.

"Eye area" denotes for example an area extending less than 5 cm, preferably less than 2 cm, from the edge of one of the eyelids 16 of one of the eyes, and preferably from the edge of the upper eyelid 16 of one of the user's eyes.

In the example in figure 1, the body area therefore comprises a movable area corresponding to the upper eyelid 16 and a stationary area corresponding to the outer corner 14 of the eye.

Alternatively, the body area 12 comprises for example the user's skin on the arm, torso, face and/or neck. In a further alternative embodiment, the body area 12 comprises for example the user's nails.

As shown in figure 2, the cosmetic article 10A comprises a decorative layer 18 and an adhesive layer 20.

Additionally, not shown, the cosmetic article 10A further includes a protective layer of the adhesive layer 20, the protective layer being applied on the adhesive layer 20 to serve as a substrate for the cosmetic article when it is not applied on a body area of a user.

Each layer 18, 20 has a closed external contour 22 delimiting a total surface area of the layer 18, 20.

Each closed external contour 22 is in particular defined perpendicularly to the thickness of the cosmetic article 10A, when the article 10A is arranged in a planar manner.

For example, the closed external contours 22 of the layers 18, 20 match and are overlaid.

The closed external contour 22 of the decorative layer 18 has a shape drawing a predetermined esthetic pattern.

Preferably, in the example in figure 1, the predetermined esthetic pattern is elongated displaying a tip. The cosmetic article 10A is then adapted to decorate the upper eyelid 16 of one of the user's eyes and form a comma at the outer corner 14 of one of the user's eyes.

The decorative layer 18 is formed of a textile material.

The textile material includes a plurality of yarns.

The textile material is for example a woven, knitted, non-woven fabric, embroidery, lace, tulle, or other.

In the example in figure 1, the textile material is a woven fabric which includes interlacing of warp yarns and weft yarns. The textile material is in particular herein a knitted fabric composed of interlacing of yarns which form stitches distributed in columns (lengthwise in the knitted fabric) and in rows (widthwise in the knitted fabric).

The textile material comprises through gaps delimited between at least two adjacent yarns. The gaps are not shown in figures 1 and 2.

Each through gap has a maximum dimension.

The maximum dimension of each through gap is preferably less than or equal to 5 times the diameter of the yarns, advantageously less than or equal to 3 times the diameter of the yarns, more preferably less than or equal to the diameter of the yarns.

The maximum dimension of each through gap is particularly dependent on the yarn density in the textile material, the shape of the yarn cross-section, the shape of the yarn surface.

The yarns of the textile material are for example, mono- or multifilament, and made of natural material, such as cotton, hemp, linen or other, or of synthetic material, such as polyamide, polyester (PES/PET), polypropylene (PP) or other.

According to an advantageous alternative embodiment, the textile material comprises at least one elastic fiber, such as elastane or elastic PBD or other.

Preferably, the textile material comprises between 2% and 20% elastane.

The decorative layer 18 has a thickness between 0.1 mm and 0.8 mm, more preferably between 0.2 mm and 0.6 mm, preferably between 0.35 mm and 0.45 mm.

The decorative layer 18 has a mass density less than 100 g/m², more preferably less than 50 g/m², and preferably less than 10 g/m².

The decorative layer 18 also has through openings 24, described in more detail hereinafter.

The through openings 24 are formed in a predetermined manner during the weaving of the textile material.

They are in particular to be differentiated from the gaps of the textile material.

The adhesive layer 20, seen in figure 2, is applied on one side of the decorative layer 18.

The adhesive layer 20 is formed by a biocompatible adhesive.

"Biocompatible adhesive" denotes according to the invention any compound which has the property of adhering to a biological tissue when said compound is applied thereon, such as for example a mucous membrane or the dermis. In order to be biocompatible, the adhesive agent must be compatible with use on biological tissue, without causing adverse reactions such as inflammation of the biological tissue.

Preferably, the biocompatible adhesive comprises silicone, in particular a silicone gel.

Such an adhesive particularly ensures a satisfactory hold over time, as well as good repositionability of the cosmetic article on the skin.

The repositionability reflects the ability of the cosmetic article to be positioned, detached, then repositioned a certain number of times on the skin, without the adhesive layer losing the adhesion capacity thereof, in other words the adhesion capacity of the adhesive layer remains sufficiently high to ensure a satisfactory hold over time even after undergoing several adhesion/detachment cycles on the skin.

For example, the biocompatible adhesive is selected from Silpuran^{®} 2114, Silpuran^{®} 2142, sold for example by WACKER, Silbione^{®} 4743 or Silbione^{®} 4645, sold for example by ELKEM, "Nero/black" silicone gel or other.

The adhesive layer 20 has a mass density between 20 g/m² and 220 g/m², and preferably between 40 g/m² and 60 g/m², for example approximately 50 or 55 g/m².

The adhesive layer 20 and the decorative layer 18 exhibit together a permeability to air, measured for example as per the ISO 9237-2 standard, of at least 3000 l/min.dm², more preferably at least 10000 l/min.dm², for example of the order of 50000 l/min.dm².

The cosmetic article formed of the adhesive layer 20 and the decorative layer 18 thus has a good skin breathability when positioned thereon.

The adhesive layer 20 also has through openings 26.

Each through opening 24, 26 of the decorative layer 18 and the adhesive layer 20 has a closed transverse internal contour.

The internal contour of each through opening 24, 26 is in particular defined perpendicularly to the thickness of the article 10A, when the article 10A is arranged in a planar manner.

In the example in figure 1, the transverse internal contour of each opening 24, 26 has a rectangular, particularly square, shape. Alternatively, the transverse internal contour of each opening 24, 26 has a polygonal, regular polygonal, parallelogram, elongated rectangular, lozenge, oval or circular shape.

In the embodiment example illustrated in figures 1 and 2, all the openings 24, 26 are substantially identical in shape and in dimensions. Alternatively, the openings 24, 26 could be different from one another, in shape and/or in dimensions.

For each through opening 24, 26, the transverse internal contour of the opening delimits an internal area of the opening.

In the embodiment example in figures 1 and 2, the internal areas defined by the through openings 24, 26 of the layers 18, 20 are substantially identical.

"Substantially identical" denotes that each internal area exhibits a deviation less than 10% from the mean value of the internal areas of the through openings 24, 26 of the layers 18, 20.

In the example in figure 1, for each layer 18, 20, the through openings of the layer form a regular grid.

More specifically, for each layer 18, 20, the through openings of the layer are distributed in a grid of rows and columns, the rows being parallel pairwise, the columns being parallel pairwise.

Furthermore, for each through opening 24, 26, the transverse internal contour of the opening has a greater dimension.

Advantageously, said greater dimension is greater than 0.5 mm.

Said greater dimension is also preferably less than 10 mm, more preferably less than 5 mm, even more preferably less than 2 mm, and in particular approximately 1 mm.

As mentioned above, the through openings 24 of the decorative layer 18 are to be differentiated from the through gaps of the textile wherein this decorative layer 18 is formed. In particular, the greater dimension of the through openings 24 is considerably greater, for example 5 times greater, more preferably 10 times greater, even more preferably 50 times greater, than the greater dimension of the through gaps.

As illustrated in figure 2, at least some of the through openings 26 of the adhesive layer 20 are respectively facing the through openings 24 of the decorative layer 18.

In projection in a plane perpendicular to the thickness, when the article 10A is arranged in a planar manner, for each through opening 24 of the decorative layer 18, the surface of the adhesive layer 20 projecting into the internal contour of said through opening 24 is less than 10% of the internal area of said opening 24.

Preferably, for each through opening 24 of the decorative layer 18, the internal contour of one of the openings 26 of the adhesive layer 20 is overlaid substantially on the internal contour of the opening 24 of the decorative layer 18.

In other words, in projection in a plane perpendicular to the thickness, when the article 10A is arranged in a planar manner, the adhesive layer 20 does not project into the internal contour of the through openings 24 of the decorative layer 18.

In particular, the adhesive layer 20 does not occlude the through openings 24 of the decorative layer 18.

Advantageously, the adhesive layer 20 only extends substantially square with the yarns of the textile material of the decorative layer 18. In a more advantageous alternative embodiment, the adhesive layer 20 covers the through gaps of the decorative layer 18.

Preferably, the decorative layer 18 and the adhesive layer 20 comprise respectively the same number of through openings 24, 26.

Advantageously, the decorative layer 18 and the adhesive layer 20 respectively comprise a number of through openings 24, 26 per dm² between 500 and 10000, and respectively have an opening rate between 20% and 95%.

The opening rate of each layer 18, 20 is defined as the ratio of the open surface of the layer to the total surface area of the layer.

The open surface area of the adhesive layer 20 is the difference between the total surface area of the adhesive layer 20 and the filled surface area of adhesive, in projection in a plane perpendicular to the thickness when the article 10A is arranged in a planar manner. This ratio defines a porosity rate (%).

The open surface area of the adhesive layer 20 is the sum of the internal areas of the through openings 26 of the adhesive layer 20.

Similarly, the open surface area of the decorative layer 18 is the difference between the total surface area of the decorative layer 18 and the filled surface area of the textile material, in projection in a plane perpendicular to the thickness when the article 10A is arranged in a planar manner.

The open surface area of the decorative layer 18 is the sum of the internal areas of the through openings 24 of the decorative layer 18.

Advantageously, the decorative layer 18 comprises a number of through openings 24 per dm² greater than or equal to 1500, for example between 1500 and 10000, and preferably between 1500 and 5500, in particular approximately 4000.

Advantageously, the adhesive layer 20 comprises a number of through openings 26 per dm² greater than or equal to 1500, for example between 1500 and 10000, and preferably between 1500 and 5500, in particular approximately 4000.

Advantageously, the decorative layer 18 has an opening rate greater than 50%, for example between 50% and 95%, and preferably between 80% and 95%.

Advantageously, the decorative layer 20 has an opening rate greater than 50%, for example between 50% and 95%, and preferably between 80% and 95%.

In a preferred embodiment, the decorative layer 18 and the adhesive layer 20 each comprise respectively a number of through openings 24, 26 per dm² greater than or equal to 1500, for example between 1500 and 10000, and preferably between 1500 and 5500, in particular approximately 4000 and each have respectively an opening rate greater than 50%, for example between 50% and 95%, and preferably between 80% and 95%.

The cosmetic article 10A furthermore has further features defined hereinafter.

In particular, the cosmetic article 10A advantageously exhibits a mean force required to detach the article 10A after the first application on a substrate, between 0.4N and 1.2N, and preferably between 0.4N and 1N.

Moreover, the cosmetic article 10A advantageously exhibits a mean force required to detach the article 10A after 10 applications on a substrate greater than or equal to 0.16N, more preferably greater than or equal to 0.2N, in particular between 0.2N and 0.6N, preferably between 0.2N and 0.5N.

This force is for example measured with the measurement protocol of the test entitled "peel test" defined hereinafter.

Such force value ranges help justify a good hold, while being sufficiently low so that the peeling of the article 10A applied on the body area 12, in particular on the skin, is not unpleasant for the user.

Advantageously, the cosmetic article 10A exhibits a mean force required to detach the article 10A after 10 applications on a substrate greater than or equal to 40%, more preferably greater than or equal to 50% of the mean force required to detach the article 10A after the first application on the substrate.

Such a ratio between the mean force required to detach the cosmetic article after the first application on a substrate and the mean force required to detach the cosmetic article after 10 applications on a substrate makes it possible to ensure good repositionability of the cosmetic article on the skin. As mentioned above, the repositionability reflects the ability of the cosmetic article to be positioned, detached, then repositioned a certain number of times on the skin, without the adhesive layer losing the adhesion capacity thereof, in other words the adhesion capacity of the adhesive layer remains sufficiently high to ensure a satisfactory hold over time even after undergoing several adhesion/detachment cycles on the skin.

The cosmetic article 10A exhibits a rupture strength, or maximum force at rupture, between 100 N and 400 N, preferably between 150 N and 250 N, advantageously between 175 N and 230 N.

In this embodiment, the cosmetic article 10A exhibits a tensile strength Rm between 16 MPa and 67 MPa, preferably between 25 MPa and 42 MPa, advantageously between 29 MPa and 39 MPa.

The cosmetic article 10A exhibits an elongation at break preferably between 100% and 160%, advantageously between 110% and 150%.

Such features make it possible in particular to enhance the flexibility of the cosmetic article and thus the comfort thereof when worn.

The rupture strength and the elongation at break are for example measured with the measurement protocol of the test entitled "pull test" defined hereinafter.

The cosmetic article 10A exhibits a shear rigidity C between 0.1 N/(m.°) and 4 N/(m.°), advantageously between 0.1 N/(m.°) and 2 N/(m.°), preferably between 0.2 N/(m.°) and 1 N/(m.°).

The cosmetic article 10A thus exhibits a satisfactory flexibility for the user when worn.

The shear force C is for example measured with the measurement protocol of the test entitled "shear test" defined hereinafter.

The cosmetic article 10A exhibits a flexural rigidity B between 0.1 µN.m²/m and 80 µN.m²/m, advantageously between 0.1 µN.m²/m and 5 µN.m²/m, preferably between 0.5 µN.m²/m and 2 µN.m²/m.

The cosmetic article 10A thus exhibits a satisfactory flexibility for the user when worn.

The flexural rigidity B is for example measured with the measurement protocol of the test entitled "flexural test" defined hereinafter.

A cosmetic application method will now be described.

The method comprises providing the cosmetic article 10A defined above.

The cosmetic article 10A is then applied on the body area 12 of the user. In particular, the adhesive layer 20 of the article 10A is applied on said area.

In an alternative embodiment of the first embodiment, at least one of the through openings 24 of the decorative layer 18, for example a plurality thereof, has a greater dimension greater than 5 mm, preferably greater than 10 mm, more preferably greater than 20 mm or more. Such an opening is hereinafter referred to using the term "major opening".

The or each major opening is bordered, preferably surrounded, by an area of the decorative layer 18 having through openings 24 wherein the greater dimension is less than that of the major opening, and for example less than 2 mm, more preferably less than 1 mm.

Such a cosmetic article is then particularly adapted for application on an extensive body area, for example greater than 4 cm², more preferably greater than 9 cm², advantageously greater than 16 cm², preferably greater than 25 cm².

The extensive body area then advantageously comprises the user's skin on the arm, torso, face and/or neck.

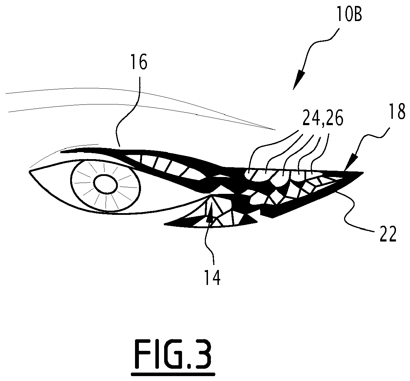

A cosmetic article 10B according to a second embodiment is illustrated in figure 3.

The cosmetic article 10B according to the second embodiment differs from the first embodiment in that the textile material of the decorative layer 18 is a lace.

"Lace" denotes a textile material with no weft or warp. A lace can also be referred to as a mixtilinear fabric.

Lace is a light and open-worked textile material, with no warp or weft, which is composed of fine stitches, interlaced yarns, so as to form a design with flowered patterns and other varied shapes and generally with serrated edges.

Lace is a textile material which exhibits very good flexibility and multiple link points. Lace therefore enhances comfort when worn, and is particularly adapted for a body area comprising movable areas such as the upper eyelid 16 of an eye.

As illustrated in figure 3, for each layer 18, 20, at least two of the internal areas of the openings of the layer are separate.

Preferably, for each layer 18, 20, at least one of the internal areas of the openings of the layer is less than 50% of one of the other internal areas of the openings of the layer.

Advantageously, for each layer 18, 20, at least two of the transverse internal contours of the openings of the layer have different shapes pairwise.

For each layer 18, 20, at least half of the transverse internal contours of the openings of the layer have different shapes pairwise.

Such separate openings enhance the link of the cosmetic article 10B on the body area 12, and therefore the comfort when worn. It is thus possible to size the shapes and the areas of the openings according to the body area intended to receive the application of the article 10B.

In the case of a body area comprising a movable area and a stationary area, it is possible to size the shapes and the areas of the openings to obtain a superior link between the part of the article 10B applied on the movable area and the part of the article 10B applied on the stationary area.

Similarly, in the case of a body area comprising a movable area and a stationary area, it is possible to size the shapes and the areas of the openings to give the part of the article 10B applied on the movable area a superior linking capability to that of the part of the article 10B applied on the stationary area.

Furthermore, the shapes of the internal contours of the openings are selected to give the cosmetic article 10B a satisfactory esthetic appearance, as illustrated in figure 3.

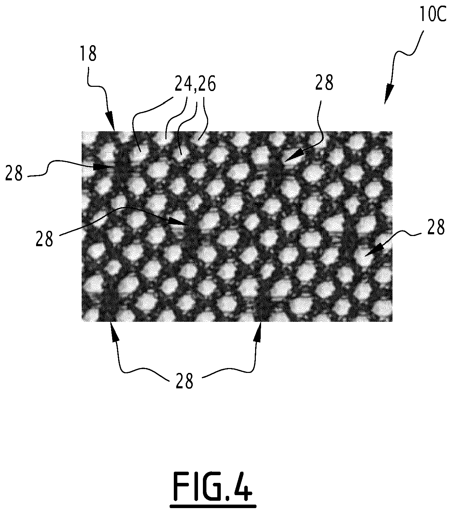

A cosmetic article 10C according to a third embodiment is illustrated in figure 4.

Figure 4 only shows a part of the cosmetic article 10C. In particular, the external contour 22 of the decorative layer 18 is not visible.

The cosmetic article 10C according to the third embodiment differs from the first embodiment in that the textile material of the decorative layer 18 is a lace.

Furthermore, the transverse internal contour of each opening 24, 26 has a hexagonal shape, preferably a regular hexagon.

In the example in figure 4, for each layer 18, 20, the through openings of the layer form a regular grid.

Furthermore, the regular grid is such that, at regular distance intervals, the decorative layer 18 has an area 28 filled with textile material instead of a through opening 24. These filled areas are distributed in staggered rows along the surface of the decorative layer 18.

Such a cosmetic article 10C is referred to in the examples hereinafter as "geometric pattern".

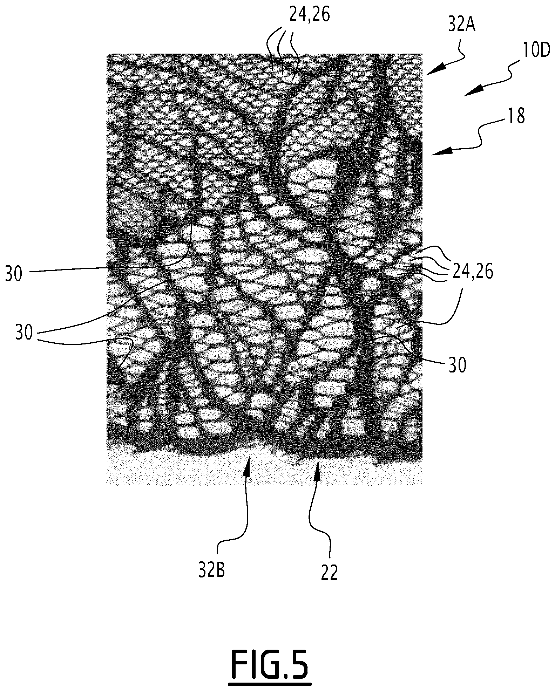

A cosmetic article 10D according to a fourth embodiment is illustrated in figure 5.

Figure 5 only shows a part of the cosmetic article 10D. In particular, the external contour 22 of the decorative layer 18 is only partially visible.

The cosmetic article 10D according to the fourth embodiment differs from the first embodiment in that the textile material of the decorative layer 18 is a lace.

As illustrated in figure 5, the decorative layer 18 comprises curved ribs 30, formed by several yarns, the ribs 30 delimiting therebetween areas having said through openings 24 of the layer 18.

The ribs 30 respectively have a width for example greater than half the maximum dimension of the through openings 24.

The decorative layer 18 has for example a central region 32A and a peripheral region 32B, the two regions 32A, 32B having different numbers of through openings 24 per dm² and/or different respective opening rates.

Each through opening 24 of the peripheral region 32B has a greater dimension greater than the respective greater dimension of each through opening 24 of the central region 32A, preferably greater than twice that of each through opening 24 of the central region 32A.

The peripheral region 32B is delimited in part by the external contour 22.

The central region 32A is for example separated from the peripheral region 32B by one of the ribs 30.

Such a cosmetic article 10D is referred to in the examples hereinafter as "flowered pattern".

### Examples:

The tests were conducted on the following samples:
- a sample, designated by the number 1, corresponding to a decorative layer of elastic knitted fabric (geometric pattern) coated with an adhesive layer of Silpuran ^{®} 2114 exhibiting a mass density of approximately 55 g/m²;
- a sample, designated by the number 2, corresponding to a decorative layer of elastic knitted fabric (geometric pattern) coated with an adhesive layer of Silbione ^{®} 4743 exhibiting a mass density of approximately 55 g/m²;
- a sample, designated by the number 3, corresponding to a decorative layer of elastic knitted fabric (geometric pattern), identical to that of sample 2, coated with an adhesive layer of Silbione ^{®} 4743 exhibiting a mass density of approximately 40 g/m²;
- a sample, designated by the number 4, corresponding to a decorative layer of elastic knitted fabric (geometric pattern), not coated with an adhesive layer;
- a sample, designated by the number 5, corresponding to a decorative layer of elastic knitted fabric (geometric pattern), different from those of samples 2, 3 and 4, coated with an adhesive layer of Silbione ^{®} 4743 exhibiting a mass density of approximately 55 g/m²;
- a sample, designated by the number 6, corresponding to a decorative layer of elastic knitted fabric (geometric pattern), different from that of sample 1, coated with an adhesive layer of Silpuran ^{®} 2114 exhibiting a mass density of approximately 55 g/m²;
- a sample, designated by the number 7, corresponding to a decorative layer of non-elastic knitted fabric (geometric pattern), not coated with an adhesive layer;
- a sample, designated by the number 8, corresponding to a decorative layer of non-elastic knitted fabric (geometric pattern) coated with an adhesive layer of "Nero/black" silicone gel exhibiting a mass density of approximately 55 g/m²;
- a sample, designated by the number 9, corresponding to a decorative layer of non-elastic knitted fabric (flowered pattern) coated with an adhesive layer of Silpuran ^{®} 2114 exhibiting a mass density of approximately 55 g/m²;
- a sample, designated by the number 10, corresponding to a decorative layer of non-elastic knitted fabric (flowered pattern) coated with an adhesive layer of Silbione ^{®} 4645 exhibiting a mass density of approximately 55 g/m²; and
- a sample, designated by the number 11, corresponding to a decorative layer of non-elastic knitted fabric (flowered pattern) coated with an adhesive layer of Silpuran ^{®} 2142 exhibiting a mass density of approximately 55 g/m².

"Elastic knitted fabric" denotes a textile material incorporating elastic fibers, such as elastane fibers.

In samples 1 to 6, the knitted fabric comprises about 15% elastic fibers, such as elastane fibers and 85% non-elastic fibers, such as polyamide fibers.

"Non-elastic knitted fabric" denotes a textile material not comprising elastic fibers, but merely non-elastic fibers, such as polyamide fibers.

The geometric pattern samples (samples 1 to 8) have a number of openings per dm² between 4800 and 5200, in particular approximately 5000, and an opening rate of approximately 85%.

The flowered pattern samples (samples 9 to 11) have a number of openings per dm² between 3800 and 4300, in particular approximately 4100, and an opening rate of approximately 91 %.

Moreover, the geometric pattern of samples 1 to 8 is illustrated in figure 4. The flowered pattern of samples 9 to 11 is illustrated in figure 5, taken entirely in the central region 32A.

### Peel test:

This property is characterized according to the principles described in the "Peel/Adherence NF EN 1372 and D11 1158" standard by amending the application and completion protocol:
- Apply the sample on a leather substrate;
- Measure the peel force after application on the leather;
- Repeat the application of the sample on the same leather 10 times and measure the peel force between each application;
- Repeat the 10 cycles 3 times; and
- Reproduce the same tests on a leather substrate having a rough/textured appearance.

The purpose of this test is to evaluate the detachment force at t=0 and after 10 applications, whether on a smooth substrate or on a textured substrate.
Test conditions: Tensile bench, Force scale: 20 N, Elongation scale: 150 mm, speed: 100 mm/min
Sample size: width: 6cm / length: 15 cm.

At t= 0, 5 cm of the sample is on top of the leather for positioning the sample in the clamping jaw.

Peeling is performed along about 9 cm (1 cm of strip remains attached to the leather between each peel test).

Then, reattach the 9 cm of the sample detached to the leather, wait one minute and repeat the test.

The peel test was used for samples 1 to 3.

### Sample 1:

**[Table 1] Results for sample 1 of the tests on a smooth leather substrate, the columns corresponding to the sample application number on the same substrate**

| **Test No.** | | **1** | **2** | **3** | **4** | **5** | **6** | **7** | **8** | **9** | **10** |
|---|---|---|---|---|---|---|---|---|---|---|---|
| **test 1** | Max force (N) | 0,946 | 0,484 | 0,36 | 0,424 | 0,434 | 0,44 | 0,426 | 0,42 | 0,366 | 0,414 |
| | Mean force (N) | 0,6791 | 0,2585 | 0,151 | 0,2105 | 0,2483 | 0,2751 | 0,2518 | 0,2299 | 0,2297 | 0,2353 |
| | Min force (N) | 0,046 | 0,054 | 0,026 | 0,054 | 0,04 | 0,086 | 0,04 | 0,014 | 0,046 | 0,046 |
| **test 2** | Max force (N) | 1,134 | 0,574 | 0,574 | 0,566 | 0,574 | 0,48 | 0,454 | 0,5 | 0,52 | 0,38 |
| | Mean force (N) | 0,8737 | 0,3887 | 0,4027 | 0,3739 | 0,3168 | 0,3315 | 0,2807 | 0,2904 | 0,2998 | 0,21 |
| | Min force (N) | 0,046 | 0,166 | 0,14 | 0,146 | 0,1 | 0,14 | 0,106 | 0,106 | 0,086 | 0,046 |
| **test 3** | Max force (N) | 1,126 | 0,686 | 0,614 | 0,58 | 0,44 | 0,554 | 0,48 | 0,49 | 0,446 | 0,406 |
| | Mean force (N) | 0,9063 | 0,5125 | 0,3999 | 0,4377 | 0,2801 | 0,3347 | 0,3191 | 0,3303 | 0,3086 | 0,2929 |
| | Min force (N) | 0,106 | 0,146 | 0,126 | 0,206 | 0,094 | 0,086 | 0,146 | 0,146 | 0,154 | 0,166 |
| **Test mean** | Max force (N) | 1,0687 | 0,5813 | 0,5160 | 0,5233 | 0,4827 | 0,4913 | 0,4533 | 0,4700 | 0,4440 | 0,4000 |
| | Mean force (N) | 0,8197 | 0,3866 | 0,3179 | 0,3407 | 0,2817 | 0,3138 | 0,2839 | 0,2835 | 0,2794 | 0,2461 |
| | Min force (N) | 0,0660 | 0,1220 | 0,973 | 0,1353 | 0,0780 | 0,1040 | 0,0973 | 0,0887 | 0,0953 | 0,0860 |

**[Table 2] Results for sample 1 of the tests on the smooth leather substrate**

| **Test No.** | | **Mean** | **Median** | **Std Dev** | **Coef Var** | **Max** | **Min** |
|---|---|---|---|---|---|---|---|
| **test 1** | Max force (N) | 0,4714 | 0,425 | 0,1705 | 36,16 | 0,946 | 0,36 |
| | Mean force (N) | 0,2769 | 0,2418 | 0,1453 | 52,5 | 0,6791 | 0,151 |
| | Min force (N) | 0,0452 | 0,046 | 0,0189 | 41,83 | 0,086 | 0,014 |
| **test 2** | Max force (N) | 0,576 | 0,543 | 0,2063 | 35,83 | 1,134 | 0,38 |
| | Mean force (N) | 0,3768 | 0,3242 | 0,1837 | 48,76 | 0,8737 | 0,21 |
| | Min force (N) | 0,1082 | 0,106 | 0,041 | 37,86 | 0,166 | 0,046 |
| **test 3** | Max force (N) | 0,5822 | 0,522 | 0,21 | 36,08 | 1,126 | 0,406 |
| | Mean force (N) | 0,4122 | 0,3325 | 0,1882 | 45,66 | 0,9063 | 0,2801 |
| | Min force (N) | 0,1376 | 0,146 | 0,036 | 26,16 | 0,206 | 0,086 |
| **Test mean** | Max force (N) | 0,5431 | 0,4870 | 0,1911 | 35,1980 | 1,0687 | 0,4000 |
| | Mean force (N) | 0,3553 | 0,2988 | 0,1678 | 47,2195 | 0,8197 | 0,2461 |
| | Min force (N) | 0,0970 | 0,0963 | 0,0202 | 20,7865 | 0,1353 | 0,0660 |

**[Table 3] Results for sample 1 of the tests on a textured leather substrate**

| **Test No.** | | **1** | **2** | **3** | **4** | **5** | **6** | **7** | **8** | **9** | **10** |
|---|---|---|---|---|---|---|---|---|---|---|---|
| **Test 1** | Max force (N) | 0,614 | 0,3 | 0,314 | 0,276 | 0,26 | 0,3 | 0,326 | 0,314 | 0,326 | 0,26 |
| | Mean force (N) | 0,4386 | 0,175 | 0,1739 | 0,1465 | 0,1534 | 0,2039 | 0,2207 | 0,2129 | 0,2084 | 0,1695 |
| | Min force (N) | 0,066 | 0,054 | 0,046 | 0,046 | 0,086 | 0,094 | 0,1 | 0,1 | 0,046 | 0,106 |
| **test 2** | Max force (N) | 0,654 | 0,426 | 0,354 | 0,34 | 0,36 | 0,314 | 0,42 | 0,34 | 0,434 | 0,41 |
| | Mean force (N) | 0,4644 | 0,2827 | 0,217 | 0,229 | 0,2416 | 0,1813 | 0,2578 | 0,2187 | 0,2623 | 0,2616 |
| | Min force (N) | 0,194 | 0,166 | 0,114 | 0,094 | 0,146 | 0,066 | 0,126 | 0,12 | 0,094 | 0,14 |
| **test 3** | Max force (N) | 0,606 | 0,32 | 0,264 | 0,18 | 0,304 | 0,27 | 0,256 | 0,264 | 0,204 | 0,314 |
| | Mean force (N) | 0,3999 | 0,2374 | 0,1769 | 0,1377 | 0,2027 | 0,1653 | 0,1836 | 0,1606 | 0,104 | 0,2024 |
| | Min force (N) | 0,134 | 0,146 | 0,1 | 0,08 | 0,1 | 0,094 | 0,134 | 0,08 | 0,026 | 0,06 |
| **Test mean** | Max force (N) | 0,6247 | 0,3487 | 0,3107 | 0,2653 | 0,3080 | 0,2947 | 0,3340 | 0,3060 | 0,3213 | 0,3280 |
| | Mean force (N) | 0,4343 | 0,2317 | 0,1893 | 0,1711 | 0,1992 | 0,1835 | 0,2207 | 0,1974 | 0,1916 | 02112, |
| | Min force (N) | 0,1313 | 0,1220 | 0,0867 | 0,0733 | 0,1107 | 0,0847 | 0,1200 | 0,1000 | 0,0553 | 0,1020 |

**[Table 4] Results for sample 1 of the tests on a textured leather substrate**

| **Test No.** | | **Mean** | **Median** | **Std Dev** | **Coef Var** | **Max** | **Min** |
|---|---|---|---|---|---|---|---|
| **test 1** | Max force (N) | 0,329 | 0,307 | 0,1031 | 31,34 | 0,614 | 0,26 |
| | Mean force (N) | 0,2103 | 0,1894 | 0,0842 | 40,05 | 0,4386 | 0,1465 |
| | Min force (N) | 0,0744 | 0,076 | 0,0252 | 33,93 | 0,106 | 0,046 |
| **test 2** | Max force (N) | 0,4052 | 0,385 | 0,0971 | 23,97 | 0,654 | 0,314 |
| | Mean force (N) | 0,2616 | 0,2497 | 0,077 | 29,42 | 0,4644 | 0,1813 |
| | Min force (N) | 0,126 | 0,123 | 0,0375 | 29,75 | 0,194 | 0,066 |
| **test 3** | Max force (N) | 0,2982 | 0,267 | 0,117 | 39,24 | 0,0606 | 0,18 |
| | Mean force (N) | 0,197 | 0,1803 | 0,0802 | 40,72 | 0,3999 | 0,104 |
| | Min force (N) | 0,0954 | 0,097 | 0,0367 | 38,48 | 0,146 | 0,026 |
| **Test mean** | Max force (N) | 0,3441 | 0,3160 | 0,1012 | 29,4017 | 0,6247 | 0,2653 |
| | Mean force (N) | 0,2230 | 0,1983 | 0,0764 | 34,2419 | 0,4343 | 0,1711 |
| | Min force (N) | 0,0986 | 0,1010 | 0,0238 | 24,1026 | 0,1313 | 0,0553 |

### Sample 2

**[Table 5] Results for sample 2 of the tests on a smooth leather substrate**

| **Test No.** | | **1** | **2** | **3** | **4** | **5** | **6** | **7** | **8** | **9** | **10** |
|---|---|---|---|---|---|---|---|---|---|---|---|
| **Test 1** | Max force (N) | 1,066 | 0,626 | 0,626 | 0,494 | 0,604 | 0,56 | 0,554 | 0,574 | 0,526 | 0,574 |
| | Mean force (N) | 0,7974 | 0,423 | 0,4391 | 0,3809 | 0,4623 | 0,4026 | 0,4132 | 0,4412 | 0,4081 | 0,4425 |
| | Min force (N) | 0,2 | 0,08 | 0,206 | 0,206 | 0,246 | 0,154 | 0,16 | 0,2 | 0,18 | 0,186 |
| **Test 2** | Max force (N) | 0,914 | 0,546 | 0,526 | 0,486 | 0,54 | 0,5 | 0,54 | 0,466 | 0,5 | 0,546 |
| | Mean force (N) | 0,7424 | 0,4431 | 0,4246 | 0,3989 | 0,4247 | 0,4129 | 0,4342 | 0,373 | 0,4189 | 0,4076 |
| | Min force (N) | 0,146 | 0,246 | 0,186 | 0,22 | 0,226 | 0,22 | 0,254 | 0,22 | 0,28 | 0,226 |
| **Test 3** | Max force (N) | 1,384 | 0,846 | 0,826 | 0,774 | 0,76 | 0,654 | 0,62 | 0,844 | 0,81 | 0,646 |
| | Mean force (N) | 1,006 | 0,589 | 0,633 | 0,594 | 0,532 | 0,509 | 0,461 | 0,582 | 0,568 | 0,478 |
| | Min force (N) | 0,194 | 0,206 | 0,26 | 0,246 | 0,2 | 0,214 | 0,18 | 0,2 | 0,174 | 0,146 |
| **Test mean** | Max force (N) | 1,1213 | 0,6727 | 0,6593 | 0,5847 | 0,6347 | 0,5713 | 0,5713 | 0,6280 | 0,6120 | 0,5887 |
| | Mean force (N) | 0,8486 | 0,4850 | 0,4989 | 0,4579 | 0,4730 | 0,4415 | 0,4361 | 0,4654 | 0,4650 | 0,4427 |
| | Min force (N) | 0,1800 | 0,1773 | 0,2173 | 0,2240 | 0,2240 | 0,1960 | 0,1980 | 0,2067 | 0,2113 | 0,1860 |

**[Table 6] Results for sample 2 of the tests on a smooth leather substrate**

| **Test No.** | | **Mean** | **Median** | **Std Dev** | **Coef Var** | **Max** | **Min** |
|---|---|---|---|---|---|---|---|
| **test 1** | Max force (N) | 0,62 | 0,574 | 0,162 | 26,11 | 1,066 | 0,494 |
| | Mean force (N) | 0,461 | 0,4311 | 0,1205 | 26,14 | 0,7974 | 0,3809 |
| | Min force (N) | 0,1818 | 0,193 | 0,0442 | 24,32 | 0,246 | 0,08 |
| **test 2** | Max force (N) | 0,556 | 0,533 | 0,1287 | 23,13 | 0,914 | 0,466 |
| | Mean force (N) | 0,448 | 0,4217 | 0,1053 | 23,49 | 0,7424 | 0,373 |
| | Min force (N) | 0,2224 | 0,223 | 0,0366 | 16,47 | 0,28 | 0,146 |
| **test 3** | Max force (N) | 0,816 | 0,792 | 0,2168 | 26,55 | 1,384 | 0,62 |
| | Mean force (N) | 0,595 | 0,575 | 0,1545 | 25,96 | 1,006 | 0,461 |
| | Min force (N) | 0,202 | 0,2 | 0,0332 | 16,45 | 0,26 | 0,146 |
| **Test mean** | Max force (N) | 0,6644 | 0,6200 | 0,1644 | 24,7445 | 1,1213 | 0,5713 |
| | Mean force (N) | 0,5014 | 0,4652 | 0,1236 | 24,6446 | 0,8486 | 0,4361 |
| | Min force (N) | 0,2021 | 0,2023 | 0,0174 | 8,5913 | 0,2240 | 0,1773 |

**[Table 7] Results for sample 2 of the tests on a textured leather substrate**

| **Test No.** | | **1** | **2** | **3** | **4** | **5** | **6** | **7** | **8** | **9** | **10** |
|---|---|---|---|---|---|---|---|---|---|---|---|
| **test 1** | Max force (N) | 0,84 | 0,554 | 0,474 | 0,534 | 0,526 | 0,434 | 0,536 | 0,44 | 0,566 | 0,414 |
| | Mean force (N) | 0,5921 | 0,3097 | 0,2892 | 0,3142 | 0,3224 | 0,2743 | 0,3224 | 0,2986 | 0,3667 | 0,2613 |
| | Min force (N) | 0,22 | 0,16 | 0,086 | 0,12 | 0,174 | 0,146 | 0,166 | 0,12 | 0,166 | 0,1 |
| **test 2** | Max force (N) | 0,92 | 0,5 | 0,474 | 0,434 | 0,506 | 0,486 | 0,54 | 0,446 | 0,38 | 0,494 |
| | Mean force (N) | 0,547 | 0,2905 | 0,3034 | 0,2741 | 0,3008 | 0,2608 | 0,2655 | 0,2874 | 0,2337 | 0,2996 |
| | Min force (N) | 0,094 | 0,114 | 0,086 | 0,086 | 0,1 | 0,034 | 0,046 | 0,074 | 0,08 | 0,06 |
| **test 3** | Max force (N) | 0,92 | 0,354 | 0,28 | 0,514 | 0,5 | 0,6 | 0,406 | 0,5 | 0,49 | 0,434 |
| | Mean force (N) | 0,6468 | 0,2057 | 0,2033 | 0,311 | 0,3039 | 0,3561 | 0,2799 | 0,3022 | 0,2753 | 0,2591 |
| | Min force (N) | 0,146 | 0,094 | 0,1 | 0,094 | 0,08 | 0,086 | 0,08 | 0,14 | 0,1 | 0,086 |
| **Test mean** | Max force (N) | 0,8933 | 0,4693 | 0,4093 | 0,4940 | 0,5107 | 0,5067 | 0,4940 | 0,4620 | 0,4787 | 0,4473 |
| | Mean force (N) | 0,5953 | 0,2686 | 0,2653 | 0,2998 | 0,3090 | 0,2971 | 0,2893 | 0,2961 | 0,2919 | 0,2733 |
| | Min force (N) | 0,1533 | 0,1227 | 0,0907 | 0,1000 | 0,1180 | 0,0887 | 0,0973 | 0,1113 | 0,1153 | 0,0820 |

**[Table 8] Results for sample 2 of the tests on the textured leather substrate:**

| **Test No.** | | **Mean** | **Median** | **Std Dev** | **Coef Var** | **Max** | **Min** |
|---|---|---|---|---|---|---|---|
| **test 1** | Max force (N) | 0,532 | 0,53 | 0,1211 | 22,77 | 0,84 | 0,414 |
| | Mean force (N) | 0,3351 | 0,312 | 0,0949 | 28,31 | 0,5921 | 0,2613 |
| | Min force (N) | 0,1458 | 0,153 | 0,0399 | 27,36 | 0,22 | 0,086 |
| **test 2** | Max force (N) | 0,518 | 0,49 | 0,1481 | 28,59 | 0,92 | 0,38 |
| | Mean force (N) | 0,3063 | 0,2889 | 0,0874 | 28,52 | 0,547 | 0,2337 |
| | Min force (N) | 0,0774 | 0,083 | 0,0246 | 31,84 | 0,114 | 0,034 |
| **test 3** | Max force (N) | 0,4998 | 0,495 | 0,1731 | 34,63 | 0,92 | 0,28 |
| | Mean force (N) | 0,3143 | 0,291 | 0,1258 | 40,02 | 0,6468 | 0,2033 |
| | Min force (N) | 0,1006 | 0,094 | 0,0235 | 23,37 | 0,146 | 0,08 |
| **Test mean** | Max force (N) | 0,5165 | 0,4863 | 0,1358 | 26,2959 | 0,8933 | 0,4093 |
| | Mean force (N) | 0,3186 | 0,2940 | 0,0983 | 30,8524 | 0,5953 | 0,2653 |
| | Min force (N) | 0,1079 | 0,1057 | 0,0210 | 19,4419 | 0,1533 | 0,0820 |

### Sample 3

**[Table 9] Results for sample 3 of the tests on a smooth leather substrate:**

| **Test No.** | | **1** | **2** | **3** | **4** | **5** | **6** | **7** | **8** | **9** | **10** |
|---|---|---|---|---|---|---|---|---|---|---|---|
| **test 1** | Max force (N) | 0,74 | 0,454 | 0,406 | 0,454 | 0,366 | 0,34 | 0,41 | 0,426 | 0,446 | 0,386 |
| | Mean force (N) | 0,473 | 0,3046 | 0,281 | 0,291 | 0,2254 | 0,2399 | 0,2723 | 0,274 | 0,3296 | 0,2657 |
| | Min force (N) | 0,12 | 0,126 | 0,08 | 0,094 | 0,074 | 0,08 | 0,114 | 0,1 | 0,126 | 0,1 |
| **Test 2** | Max force (N) | 0,8 | 0,446 | 0,526 | 0,486 | 0,514 | 0,426 | 0,486 | - | 0,446 | 0,426 |
| | Mean force (N) | 0,5339 | 0,3063 | 0,3419 | 0,3278 | 0,3618 | 0,2794 | 0,3241 | - | 0,313 | 0,2855 |
| | Min force (N) | 0,126 | 0,114 | 0,086 | 0,14 | 0,146 | 0,114 | 0,126 | - | 0,1 | 0,1 |
| **Test mean** | Max force (N) | 0,7700 | 0,4500 | 0,4660 | 0,4700 | 0,4400 | 0,3830 | 0,4480 | 0,4260 | 0,4460 | 0,4060 |
| | Mean force (N) | 0,5035 | 0,3055 | 0,3115 | 0,3094 | 0,2936 | 0,2597 | 0,2982 | 0,2740 | 0,3213 | 0,2756 |
| | Min force (N) | 0,1230 | 0,1200 | 0,0830 | 0,1170 | 0,1100 | 0,0970 | 0,1200 | 0,1000 | 0,1130 | 0,1000 |

**[Table 10] Results for sample 3 of the tests on the smooth leather substrate:**

| **Test No.** | | **Mean** | **Median** | **Std Dev** | **Coef Var** | **Max** | **Min** |
|---|---|---|---|---|---|---|---|
| **test 1** | Max force (N) | 0,4428 | 0,418 | 0,1111 | 25,08 | 0,74 | 0,34 |
| | Mean force (N) | 0,2956 | 0,2775 | 0,069 | 23,35 | 0,473 | 0,2254 |
| | Min force (N) | 0,1014 | 0,1 | 0,0196 | 19,28 | 0,126 | 0,074 |
| **test 2** | Max force (N) | 0,5002 | 0,486 | 0,116 | 23,20 | 0,8 | 0,426 |
| | Mean force (N) | 0,3387 | 0,3241 | 0,0766 | 22,62 | 0,5339 | 0,2794 |
| | Min force (N) | 0,1152 | 0,114 | 0,0196 | 17,06 | 0,146 | 0,086 |
| **Test mean** | Max force (N) | 0,4705 | 0,4470 | 0,1085 | 23,0632 | 0,7700 | 0,3830 |
| | Mean force (N) | 0,3152 | 0,3018 | 0,0689 | 21,8585 | 0,5035 | 0,2597 |
| | Min force (N) | 0,1083 | 0,1115 | 0,0129 | 11,9050 | 0,1230 | 0,0830 |

**[Table 11] Results for sample 3 of the tests on a textured leather substrate**

| **Test No.** | | **1** | **2** | **3** | **4** | **5** | **6** | **7** | **8** | **9** | **10** |
|---|---|---|---|---|---|---|---|---|---|---|---|
| **test 1** | Max force (N) | 0,66 | 0,346 | 0,32 | 0,3 | 0,254 | 0,3 | 0,3 | 0,36 | 0,3 | 0,286 |
| | Mean force (N) | 0,3746 | 0,2242 | 0,1939 | 0,1906 | 0,1744 | 0,1894 | 0,1921 | 0,2158 | 0,1957 | 0,1794 |
| | Min force (N) | 0,114 | 0,08 | 0,074 | 0,08 | 0,08 | 0,094 | 0,074 | 0,094 | 0,086 | 0,074 |
| **test 2** | Max force (N) | 0,746 | 0,414 | 0,414 | 0,5 | 0,406 | 0,386 | 0,454 | 0,426 | 0,36 | 0,346 |
| | Mean force (N) | 0,4565 | 0,248 | 0,2587 | 0,3272 | 0,2632 | 0,2337 | 0,3047 | 0,3092 | 0,2186 | 0,2575 |
| | Min force (N) | 0,14 | 0,106 | 0,08 | 0,12 | 0,114 | 0,066 | 0,1 | 0,126 | 0,1 | 0,094 |
| **Test mean** | Max force (N) | 0,7030 | 0,3800 | 0,3670 | 0,4000 | 0,3300 | 0,3430 | 0,3770 | 0,3930 | 0,3300 | 0,3160 |
| | Mean force (N) | 0,4156 | 0,2361 | 0,2263 | 0,2589 | 0,2188 | 0,2116 | 0,2484 | 0,2625 | 0,2072 | 0,2185 |
| | Min force (N) | 0,1270 | 0,0930 | 0,0770 | 0,1000 | 0,0970 | 0,0800 | 0,0870 | 0,1100 | 0,0930 | 0,0840 |

**[Table 12] Results for sample 3 of the tests on the textured leather substrate**

| **Test No.** | | **Mean** | **Median** | **Std Dev** | **Coef Var** | **Max** | **Min** |
|---|---|---|---|---|---|---|---|
| **test 1** | Max force (N) | 0,3426 | 0,3 | 0,1154 | 33,69 | 0,65 | 0,254 |
| | Mean force (N) | 0,213 | 0,193 | 0,0587 | 27,57 | 0,3746 | 0,1744 |
| | Min force (N) | 0,085 | 0,08 | 0,0127 | 14,89 | 0,114 | 0,074 |
| **Test 2** | Max force (N) | 0,4452 | 0,414 | 0,1145 | 25,72 | 0,746 | 0,346 |
| | Mean force (N) | 0,2877 | 0,261 | 0,0687 | 23,86 | 0,4565 | 0,2186 |
| | Min force (N) | 0,1046 | 0,103 | 0,0218 | 20,87 | 0,14 | 0,066 |
| **Test mean** | Max force (N) | 0,3939 | 0,3720 | 0,1124 | 28,5232 | 0,7030 | 0,3160 |
| | Mean force (N) | 0,2504 | 0,2312 | 0,0612 | 24,4339 | 0,4156 | 0,2072 |
| | Min force (N) | 0,0948 | 0,0930 | 0,0150 | 15,8017 | 0,1270 | 0,0770 |

### Pull test:

The standard applied is the standard NF ISO 5081: Textiles - Woven fabrics - Determination of breaking strength and elongation (Strip method).

The test conditions are as follows: tensile bench, Force scale: 200 N, speed: 50 mm/min,
Sample size: width: 6cm / length: 15 cm.
Sample distance between clamping jaws: 10 cm

### Shear and flexural test

These two tests were carried out using two modules provided in the Kawabata (KES-f) evaluation system developed by Professor Kawabata to be able to avail of instruments for characterizing the physical and surface properties of the textiles under low stress, and thus express the value of the total hand, in other words the texture of the textiles.

This evaluation system is considered as a reference in the textile field, and it has been described in several publications.

The first version of Kawabata (KES-F) was created in 1972, modifications were made to this system by Kato Tekko Co, Kyoto, Japan, in conjunction with Kawabata.

The Kawabata chain comprises 5 different modules which measure cloth properties: KES-FB1: Tensile and shear module, KES-FB2: Flexural module, KES-FB3: Compression module, KES-FB4: Surface condition module: friction, roughness and KES-FB7: Thermal module.

### Shear test

This test is carried out by the KES-FB1 tensile and shear module.

The sample is placed flat between two horizontal clamps at a distance of 5 cm from one another. The effective size of the sample is then 20 cm wide and 5 cm long.

The clamping is 10 cm.kg.

During the shear test, the rear clamp moves parallel with the front clamp that a suitable mechanism releases so that it can rotate freely about a longitudinal axis.

A weighing bar is coupled with this free clamp on the axis thereof, so as to keep the sample at a constant tension throughout the test. The tensile weight is 200g.

The deformation is performed at a constant speed of 0.417 mm/s on either side of the initial position at rest where warp yarns and weft yarns form a theoretical angle of 90 degrees. The deformation is generally performed at + and - 8 degrees on either side of this position but can be set as desired between 0 and 8 degrees.

One cycle = rest → positive shear → rest → negative shear → rest.

It is possible to measure 3 parameters:
G: Shear rigidity at ± 2.5 degrees in N/m.degree
2HG: Hysteresis at ± 0.5 degrees in N/m
2HG5: Hysteresis at ± 5 degrees in N/m

The values retained are the mean of the values measured during positive and negative deformations.

The test is conducted in both orthogonal directions of the sample.

**[Table 13]**

| | Shear | | |
|---|---|---|---|
| quantity | Shear rigidity G | Coefficient 2HG | Coefficient 2HG5 |
| unit | N/(m.°) | N/m | N/m |
| Sample 4 | 0.35 | 0.43 | 0.53 |
| Sample 5 | 0.30 | 0.57 | 0.76 |
| Sample 6 | 0.36 | 0.83 | 0.83 |
| Sample 7 | 0.38 | 0.65 | 1.14 |
| Sample 8 | 0.31 | 0.66 | 0.71 |
| Sample 9 | 0.35 | 0.80 | 0.90 |
| Sample 10 | 0.57 | 1.10 | 1.73 |
| Sample 11 | 0.56 | 1.11 | 1.55 |

### Flexural test:

This test is carried out by the KES-FB2 flexural module.

The sample is placed between two vertical clamps at a distance of 1 cm from one another. The effective size of the sample is then 20 cm long and 1cm wide.

The rear clamp is connected to a torsion sensor. The front clamp is rotated about the first by a special mechanism ensuring a constant curvature of deformation, that of a circle.

The deformation is performed at a constant speed of 0.5 cm⁻¹/s on either side of the initial position at rest where the curvature (1/Radius) is therefore zero.

The deformation is generally performed at + and - 2.5 cm⁻¹ of this position but can be set as desired between 0 and 2.5 cm⁻¹.

One cycle = rest → positive flexion → rest → negative flexion → rest.

It is possible to measure two parameters:
B: Flexural rigidity at + 1 cm⁻¹ of curvature expressed in µN.m²/m
2HB: Hysteresis at + 1 cm⁻¹ expressed in mN.m/m

The values retained are the mean of the values measured during positive and negative deformations.

The test is conducted in both orthogonal directions of the sample.

**[Table 14]**

| | Flexion | |
|---|---|---|
| quantity | Flexural rigidity B | Coefficient 2HB |
| unit | µN.m²/m | mN.m/m |
| Sample 4 | 0.87 | 0.05 |
| Sample 5 | 0.76 | 0.08 |
| Sample 6 | 0.87 | 0.09 |
| Sample 7 | 0.93 | 0.11 |
| Sample 8 | 0.76 | 0.07 |
| Sample 9 | 1.22 | 0.10 |
| Sample 10 | 1.34 | 0.10 |
| Sample 11 | 1.64 | 0.10 |

The shear and flexural tests above demonstrated that the cosmetic article according to the invention exhibits similar shear and flexural properties to silk which is light, soft and flexible.

Indeed, silk typically exhibits a flexural rigidity B between 0.1 µN.m²/m and 0.3 µN.m²/m, and a shear rigidity G between 0.1 N/(m.°) and 0.15 N/(m.°).

These results therefore confirm that the cosmetic article has a high level of flexibility suitable for complying with the constraints of the cosmetic sector and more particularly the constraints of products intended to be placed in contact with the skin.

In addition to having these advantageous properties of lightness, softness and flexibility, the cosmetic article also exhibits a good hold and good repositionability on the body area of the user, as demonstrated by the peel tests above.

## Claims

1. Cosmetic article (10A; 10B; 10C; 10D), intended to be applied on a body area (12) of a user, comprising a decorative layer (18) and an adhesive layer (20);
the decorative layer (18) having through openings (24) and being formed of a textile material;
the adhesive layer (20) being formed by a biocompatible adhesive, the adhesive layer (20) being applied on one side of the decorative layer (18) and having through openings (26), at least some of the through openings (26) of the adhesive layer (20) respectively facing the through openings (24) of the decorative layer (18);
the decorative layer (18) comprising a number of through openings (24) per dm² between 500 and 10000, and having an opening rate between 20% and 95%.

2. Article according to claim 1, wherein the decorative layer (18) comprises a number of through openings (24) per dm² greater than 1500, for example between 1500 and 10000, and preferably between 1500 and 5500.

3. Article according to any one of claims 1 or 2, wherein the decorative layer (18) has an opening rate greater than 50%, for example between 50% and 95%, and preferably between 80% and 95%.

4. Article according to any one of the preceding claims, wherein each through opening (24, 26) has a closed transverse internal contour, the transverse internal contour delimiting an internal area of the opening (24, 26), the internal areas defined by the through openings (24, 26) of the layers (18, 20) being substantially identical.

5. Article according to any one of the preceding claims, wherein the textile material of the decorative layer (18) is a lace.

6. Article according to any one of the preceding claims, where each through opening (24, 26) has a closed transverse internal contour, the transverse internal opening delimiting an internal area of the opening (24, 26), at least two of the internal areas being separate, and at least one of the internal areas being preferably less than 50% of one of the other internal areas.

7. Article according to any one of the preceding claims, wherein each through opening (24) of the decorative layer (18) has a closed transverse internal contour having a greater dimension, the greater dimension of each opening being greater than 0.5 mm, and preferably less than 5 mm.

8. Article according to any one of the preceding claims, wherein the biocompatible adhesive comprises silicone, preferably a silicone gel.

9. Article according to any one of the preceding claims, wherein the adhesive layer (20) has a mass density between 20 g/m² and 220 g/m², and preferably between 40 g/m² and 60 g/m².

10. Article according to any one of the preceding claims, wherein the decorative layer (18) has a thickness between 0.2 mm and 0.6 mm, preferably between 0.35 mm and 0.45 mm.

11. Article according to any one of the preceding claims, wherein the decorative layer (18) has a mass density less than 100 g/m², more preferably less than 50 g/m², and preferably less than 10 g/m².

12. Article according to any one of the preceding claims, wherein the article exhibits a force required to detach the article after 10 applications on a substrate greater than or equal to 0.16N, more preferably greater than or equal to 0.2N, in particular, between 0.2N and 0.6N, preferably between 0.2N and 0.5N.

13. Article according to any one of the preceding claims, wherein the article exhibits a flexural rigidity B between 0.1 µN.m²/m and 80 µN.m²/m, advantageously between 0.1 µN.m²/m and 5 µN.m²/m, preferably between 0.5 µN.m²/m and 2 µN.m²/m.

14. Article according to any one of the preceding claims, wherein the article exhibits a shear rigidity C between 0.1 N/(m.°) and 4 N/(m.°), advantageously between 0.1 N/(m.°) and 2 N/(m.°), preferably between 0.2 N/(m.°) and 1 N/(m.°).

15. Cosmetic application method including the following steps:
- providing a cosmetic article (10A; 10B; 10C; 10D) according to any one of the preceding claims; and
- applying the adhesive layer on a body area (12) of a user.

## Patentansprüche

1. Kosmetischer Artikel (10A; 10B; 10C; 10D), der dazu bestimmt ist, auf einen Körperbereich (12) eines Benutzers aufgetragen zu werden, mit einer Dekorschicht (18) und einer Klebeschicht (20);
wobei die Dekorschicht (18) Durchgangsöffnungen (24) aufweist und aus einem Textilmaterial besteht;
die Klebeschicht (20) durch einen biokompatiblen Klebstoff gebildet wird, wobei die Klebeschicht (20) auf einer Seite der Dekorschicht (18) aufgebracht ist und Durchgangsöffnungen (26) aufweist, wobei zumindest einige der Durchgangsöffnungen (26) der Klebeschicht (20) jeweils den Durchgangsöffnungen (24) der Dekorschicht (18) gegenüberliegen;
die Dekorschicht (18) eine Anzahl von Durchgangsöffnungen (24) pro dm² zwischen 500 und 10000 aufweist und eine Öffnungsrate zwischen 20% und 95% hat.

2. Artikel nach Anspruch 1, wobei die Dekorschicht (18) eine Anzahl von Durchgangsöffnungen (24) pro dm² aufweist, die größer ist als 1500, beispielsweise zwischen 1500 und 10000, und vorzugsweise zwischen 1500 und 5500.

3. Artikel nach einem der Ansprüche 1 oder 2, wobei die Dekorschicht (18) eine Öffnungsrate von mehr als 50 %, beispielsweise zwischen 50 % und 95 %, und vorzugsweise zwischen 80 % und 95 % aufweist.

4. Artikel nach einem der vorhergehenden Ansprüche, wobei jede Durchgangsöffnung (24, 26) eine geschlossene Querinnenkontur aufweist, wobei die Querinnenkontur eine Innenfläche der Öffnung (24, 26) begrenzt, wobei die durch die Durchgangsöffnungen (24, 26) der Schichten (18, 20) definierten Innenflächen im Wesentlichen identisch sind.

5. Artikel nach einem der vorhergehenden Ansprüche, wobei das Textilmaterial der Dekorschicht (18) eine Spitze ist.

6. Artikel nach einem der vorhergehenden Ansprüche, wobei jede Durchgangsöffnung (24, 26) eine geschlossene quer verlaufende Innenkontur aufweist, wobei die quer verlaufende Innenöffnung einen Innenbereich der Öffnung (24, 26) begrenzt, wobei mindestens zwei der Innenbereiche getrennt sind und mindestens einer der Innenbereiche vorzugsweise weniger als 50 % eines der anderen Innenbereiche beträgt.

7. Artikel nach einem der vorhergehenden Ansprüche, wobei jede Durchgangsöffnung (24) der Dekorschicht (18) eine geschlossene innere Querkontur mit einer größeren Abmessung aufweist, wobei die größere Abmessung jeder Öffnung größer als 0,5 mm und vorzugsweise kleiner als 5 mm ist.

8. Artikel nach einem der vorhergehenden Ansprüche, wobei der biokompatible Klebstoff Silikon, vorzugsweise ein Silikongel, umfasst.

9. Artikel nach einem der vorhergehenden Ansprüche, wobei die Klebeschicht (20) eine Massendichte zwischen 20 g/m² und 220 g/m², vorzugsweise zwischen 40 g/m² und 60 g/m² aufweist.

10. Artikel nach einem der vorhergehenden Ansprüche, wobei die Dekorschicht (18) eine Dicke zwischen 0,2 mm und 0,6 mm, vorzugsweise zwischen 0,35 mm und 0,45 mm aufweist.

11. Artikel nach einem der vorhergehenden Ansprüche, wobei die Dekorschicht (18) eine Massendichte von weniger als 100 g/m², möglichst weniger als 50 g/m² und vorzugsweise weniger als 10 g/m² aufweist.

12. Artikel nach einem der vorhergehenden Ansprüche, wobei der Artikel eine Kraft aufweist, die erforderlich ist, um den Artikel nach 10 Anwendungen auf einem Substrat abzulösen, die größer oder gleich 0,16 N, vorzugsweise größer oder gleich 0,2 N, insbesondere zwischen 0,2 N und 0,6 N, vorzugsweise zwischen 0,2 N und 0,5 N ist.

13. Artikel nach einem der vorhergehenden Ansprüche, wobei der Artikel eine Biegesteifigkeit B zwischen 0,1 µN.m²/m und 80 µN.m²/m, vorteilhafterweise zwischen 0,1 µN.m²/m und 5 µN.m²/m, vorzugsweise zwischen 0,5 µN.m²/m und 2 µN.m²/m aufweist.

14. Artikel nach einem der vorhergehenden Ansprüche, wobei der Artikel eine Schersteifigkeit C zwischen 0,1 N/(m.°) und 4 N/(m.°), vorteilhafterweise zwischen 0,1 N/(m.°) und 2 N/(m.°), vorzugsweise zwischen 0,2 N/(m.°) und 1 N/(m.°) aufweist.

15. Verfahren zur kosmetischen Anwendung, das die folgenden Schritte umfasst:
- Bereitstellen eines kosmetischen Artikels (10A; 10B; 10C; 10D) nach einem der vorhergehenden Ansprüche; und
- Aufbringen der Klebeschicht auf einen Körperbereich (12) eines Benutzers.

## Revendications

1. Article cosmétique (10A ; 10B ; 10C ; 10D), destiné à être appliqué sur une zone du corps (12) d'un utilisateur, comprenant une couche décorative (18) et une couche adhésive (20) ;
la couche décorative (18) présentant des ouvertures traversantes (24) et étant constituée d'un matériau textile ;
la couche adhésive (20) étant formée d'un adhésif biocompatible, la couche adhésive (20) étant appliquée sur un côté de la couche décorative (18) et présentant des ouvertures traversantes (26), au moins certaines des ouvertures traversantes (26) de la couche adhésive (20) faisant respectivement face aux ouvertures traversantes (24) de la couche décorative (18) ;
la couche décorative (18) comprenant un nombre d'ouvertures traversantes (24) par dm² compris entre 500 et 10 000, et présentant un taux d'ouverture compris entre 20 % et 95 %.

2. Article selon la revendication 1, dans lequel la couche décorative (18) comprend un nombre d'ouvertures traversantes (24) par dm² supérieur à 1 500, par exemple entre 1 500 et 10 000, et de préférence entre 1 500 et 5 500.

3. Article selon l'une quelconque des revendications 1 ou 2, dans lequel la couche décorative (18) présente un taux d'ouverture supérieur à 50 %, par exemple entre 50 % et 95 %, et de préférence entre 80 % et 95 %.

4. Article selon l'une quelconque des revendications précédentes, dans lequel chaque ouverture traversante (24, 26) présente un contour interne transversal fermé, le contour interne transversal délimitant une zone interne de l'ouverture (24, 26), les zones internes définies par les ouvertures traversantes (24, 26) des couches (18, 20) étant sensiblement identiques.

5. Article selon l'une quelconque des revendications précédentes, dans lequel le matériau textile de la couche décorative (18) est une dentelle.

6. Article selon l'une quelconque des revendications précédentes, où chaque ouverture traversante (24, 26) présente un contour interne transversal fermé, l'ouverture interne transversale délimitant une zone interne de l'ouverture (24, 26), au moins deux des zones internes étant séparées, et au moins une des zones internes étant de préférence inférieure à 50 % de l'une des autres zones internes.

7. Article selon l'une quelconque des revendications précédentes, dans lequel chaque ouverture traversante (24) de la couche décorative (18) présente un contour interne transversal fermé doté d'une plus grande dimension, la plus grande dimension de chaque ouverture étant supérieure à 0,5 mm, et de préférence inférieure à 5 mm.

8. Article selon l'une quelconque des revendications précédentes, dans lequel l'adhésif biocompatible comprend du silicone, de préférence un gel de silicone.

9. Article selon l'une quelconque des revendications précédentes, dans lequel la couche adhésive (20) présente une densité massique comprise entre 20 g/m² et 220 g/m², et de préférence entre 40 g/m² et 60 g/m².

10. Article selon l'une quelconque des revendications précédentes, dans lequel la couche décorative (18) présente une épaisseur comprise entre 0,2 mm et 0,6 mm, de préférence entre 0,35 mm et 0,45 mm.

11. Article selon l'une quelconque des revendications précédentes, dans lequel la couche décorative (18) présente une densité massique inférieure à 100 g/m², de préférence inférieure à 50 g/m², et de préférence inférieure à 10 g/m².

12. Article selon l'une quelconque des revendications précédentes, dans lequel l'article présente une force nécessaire au détachement de l'article après 10 applications sur un substrat supérieure ou égale à 0,16N, de préférence supérieure ou égale à 0,2N, en particulier, comprise entre 0,2N et 0,6N, de préférence entre 0,2N et 0,5N.

13. Article selon l'une quelconque des revendications précédentes, dans lequel l'article présente une rigidité à la flexion B comprise entre 0,1 µN.m²/m et 80 µN.m²/m, avantageusement entre 0,1 µN.m²/m et 5 µN.m²/m, de préférence entre 0,5 µN.m²/m et 2 µN.m²/m.

14. Article selon l'une quelconque des revendications précédentes, dans lequel l'article présente une rigidité au cisaillement C comprise entre 0,1 N/(m.°) et 4 N/(m.°), avantageusement entre 0,1 N/(m.°) et 2 N/(m.°), de préférence entre 0,2 N/(m.°) et 1 N/(m.°).

15. Procédé d'application cosmétique incluant les étapes suivantes :
- fournir un article cosmétique (10A ; 10B ; 10C ; 10D) selon l'une quelconque des revendications précédentes ; et
- appliquer la couche adhésive sur une partie du corps (12) d'un utilisateur.
